Europäisches Patentamt

European Patent Office

Office européen des brevets

(19)

(11) Publication number: **0 237 913**

**A1**

# EUROPEAN PATENT APPLICATION

(21) Application number: **87103385.8**

(22) Date of filing: **10.03.87**

(51) Int. Cl.4: **C12N 15/00** , C12N 1/16 ,
C12N 1/18 ,
//(C12N1/18,C12R1:865)

(30) Priority: **21.03.86 US 842501**

(43) Date of publication of application:
**23.09.87 Bulletin 87/39**

(84) Designated Contracting States:
**AT BE CH DE FR GB IT LI LU NL SE**

(71) Applicant: **MILES LABORATORIES, INC.**
**1127 Myrtle Street**
**Elkhart Indiana 46514(US)**

(72) Inventor: **Armilei, Genevieve**
**1107 Pine Street**
**Elkhart, IN 46514(US)**
Inventor: **Yarger, James Gregory**
**1539 Evergreen Place**
**Elkhart, IN 46514(US)**

(74) Representative: **Jesse, Ralf-Rüdiger, Dr. et al**
**Bayer AG Konzernverwaltung RP**
**Patentabteilung**
**D-5090 Leverkusen 1 Bayerwerk(DE)**

(54) **Transcription effector sequences.**

(57) A transcription effector sequence is provided which was isolated from an approximately 450 base pair Nco1 to Taq1 fragment of the Saccharomyces cerevisiae URA3 gene promoter region. Said transcription effector sequence may be used for enhancing the expression of a gene encoding a biologically active material.

EP 0 237 913 A1

## TRANSCRIPTION EFFECTOR SEQUENCES

### Background of the Invention

With the recent advances in recombinant DNA technology it has become possible to produce biologically active materials in host organisms in quantities which heretofore could not have been readily isolated from natural sources. Such materials include, for example, proinsulin, insulin, human or bovine growth hormone, various interferons, blood coagulation factors, chymosin and other enzymes of industrial importance, somatostatin, thymosin, various antibodies and the like. As a prerequisite to the expression and subsequent recovery of the biologically active material, a faithful messenger RNA (mRNA) transcript of the gene encoding said material must be provided so that upon translation of the mRNA at the ribosome the desired biologically active material is obtained either in its mature form or as the zymogen.

DNA transcription is initiated by the action of the enzyme RNA polymerase which binds to promoter regions of the DNA template. Once initiated, transcription of the DNA template continues via RNA polymerase through the "structural" gene (i.e., the gene encoding the biologically active material) until such point as a transcription terminator is reached and mRNA is formed. In eucaryotic cells, the mRNA is released for transport into the cytoplasm for subsequent translation. The DNA region transcribed is therefore normally considered to be bounded by promoter and terminator regions which are not necessarily transcribed but which are intimately involved in the accurate synthesis of the mRNA molecule.

Conventional wisdom has suggested that transcription terminators must be present "downstream" of the structural gene encoding the biologically active material (i.e., toward the 3' end of the gene) in order for efficient transcription to occur. For example, in Cell, Vol. 28, pp. 563-573 (March 1982) is described what the authors suggest may be a consensus sequence for the termination of transcription in the yeast Saccharomyces cerevisiae. The postulated consensus sequence of TAG...TAGT...(A-T rich)...TTT is said to cause transcription to efficiently terminate a short distance downstream thereof.

In Proc. Natl. Acad. Sci. USA, Vol. 76, No. 12, pp. 6186-6190 (December 1979) the authors describe the presence of transcription terminators in certain bacterial leader sequences. These terminators are believed to be a part of a transcription attenuation system in which transcription terminates at the attenuator under conditions of amino acid surplus, i.e., trp, phe, his, thr, and leu. However, during amino acid deficiency, transcription continues past the attenuator and the gene is expressed.

In European Patent Application 154566, DNA segments obtained by Hind III digestion of certain papovirus BK mutants are described. The resulting DNA segments are utilized in conjunction with a gene encoding the protein material of interest to transform a eucaryotic host cell, specifically mouse fibroblast L-(TK⁻) cells. The production of said protein material is said to be increased manifold times than if said DNA segments were not used.

European Patent Application 164556 describes hybrid promoters composed of the RNA polymerase binding region of a yeast glycolytic enzyme promoter and a region upstream of the polymerase binding region which differs from the wild-type upstream region of said RNA polymerase region. These promoters are said to be useful for enhanced efficiency of transcription.

Research which led to the present invention focused on a detailed analysis of the URA3 promoter region from the yeast Saccharomyces cerevisiae. A cloned fragment of the yeast URA3 promoter region contains a sequence of DNA capable of functioning as a highly efficient transcription terminator. This sequence of DNA is less than or equal to 110 base pairs in length and resides between bases 45 and 155 upstream of the URA3 primary ATG codon which is located at base 227. Because this region is located within the URA3 promoter region rather than downstream of the URA3 coding region, it would be a misnomer to refer to it as a "transcription terminator" as that phrase is used conventionally. This region is more properly referred to as a transcription effector sequence. Said transcription effector sequence provides a portable, highly efficient yeast transcription terminator which functions independently of its orientation to the structural gene of interest and can easily be inserted downstream of the gene encoding the chosen biologically active material. The transcription effector sequence, when placed 3' to the gene of interest, provides for enhanced expression of the gene encoding the chosen biologically active material by virtue of its ability to efficiently terminate transcription.

Additionally, the transcription effector sequence can be readily placed upstream of the gene of interest encoding the biologically active material to enhance the expression of said gene by providing both an efficient transcription terminator to prevent expression problems related to low level read-through transcription and by providing an upstream activator binding site from the URA3 gene.

## Summary of the Invention

The present invention is directed to a DNA segment encoding a transcription effector sequence comprising an approximately 110 base pair region of the yeast URA3 promoter region residing between bases 45 and 155 upstream of the URA3 primary transcription initiation codon located at base 227, and functional subunits and derivatives thereof.

Also disclosed is a method for enhancing the expression of a gene encoding a biologically active material. Said method comprises the steps of: (a) introducing the transcription effector sequence of the present invention into a vector containing the gene encoding the biologically active material thereby creating a recombinant vector; (b) transforming a suitable host cell with the recombinant vector of step (a); and (c) culturing said transformed host cell to effect expression of said biologically active material. Expression vectors containing the transcription effector sequence and a gene encoding a biologically active material are also disclosed.

## Brief Description of the Drawings

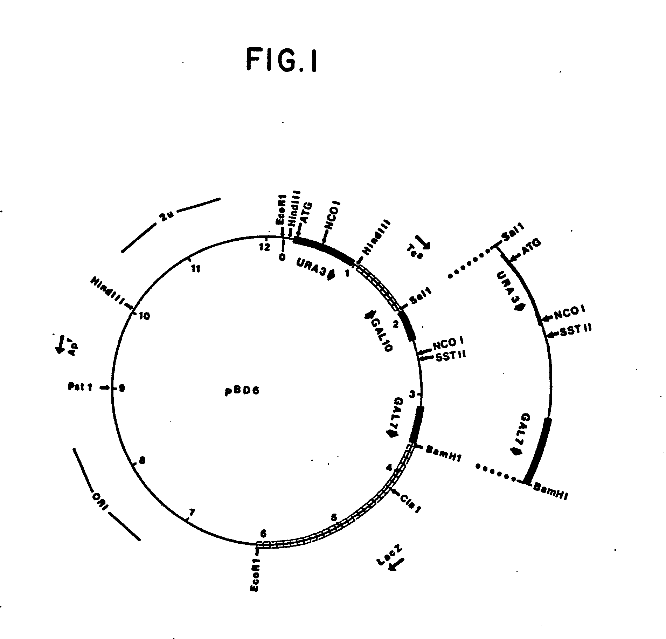

Figure 1 shows the structure of plasmids pBD6 and pBD6-R (pBD6-R is denoted by the insert shown between the Sal1 and BamH1 sites). Plasmid PBD6 contains an insertionally inactivated tetracycline gene - (Tc$^s$) with the yeast URA3 gene located within the promoter region thereof; an ampicillin resistance gene - (Ap$^r$); a bacterial origin of replication (Ori); and the B fragment of the yeast 2$\mu$ replicon (2$\mu$). The amino terminal portion of the yeast GAL7 gene is fused in-frame to the E. coli LacZ gene, and GAL10 denotes the carboxyl terminus of the yeast GAL10 gene.

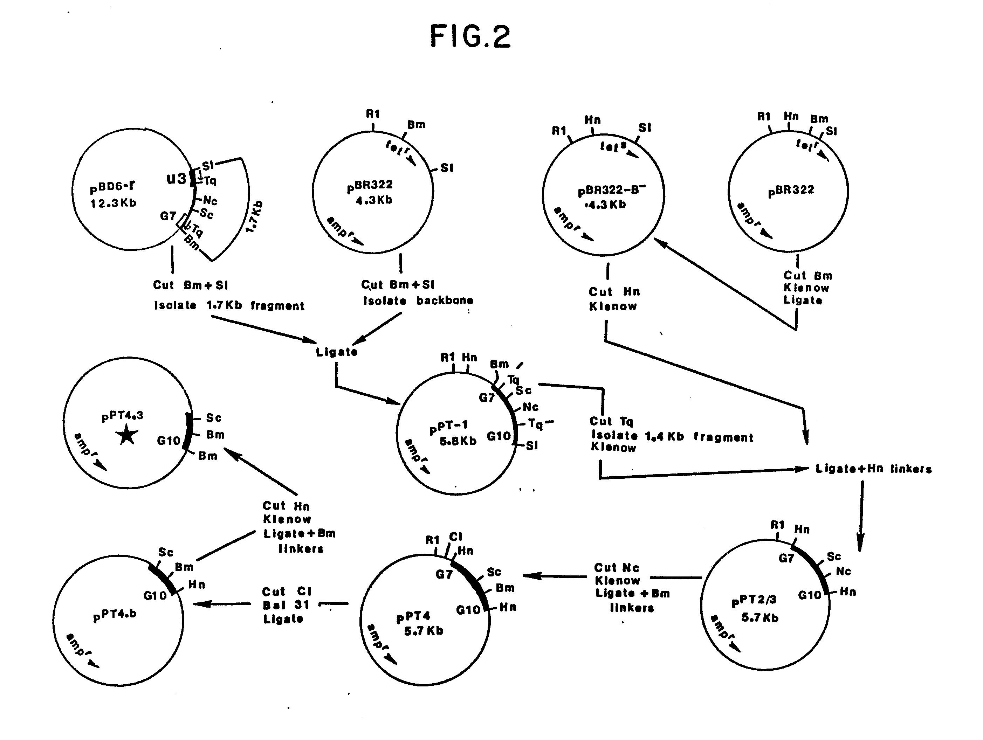

Figure 2 graphically illustrates the isolation of the 437 base pair Taq1 to Nco1 amino terminal fragment of the URA3 gene of Saccharomyces cerevisiae containing the transcription effector sequence of the present invention. The following abbreviations in Figure 2 refer to the following restriction sites: Bm (BamH1); Cl (Cla1 ); R1 (EcoR1); Hn (HindIII); Nc (Nco1); S1 (Sal1); Sc (SacII); Tq (Taq1). Plasmid pPT4.3 (denoted by star) contains said 437 base pair fragment bounded by BamH1 linkers. The abbreviations G7 and U3 refer to the GAL7 gene segment and the URA3 gene segment (in pBD6-R).

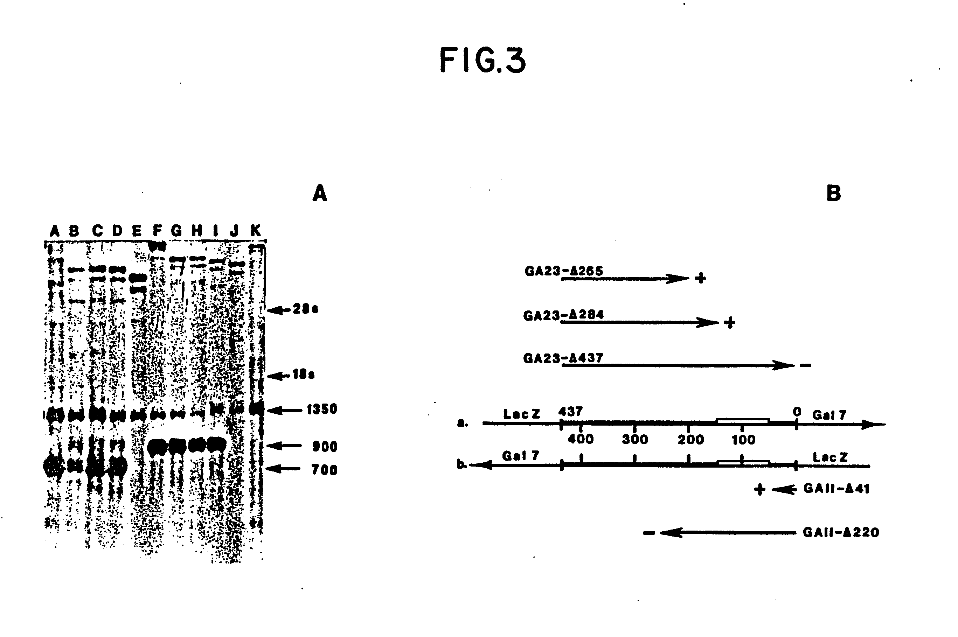

Figure 3 denotes the results of Bal31 deletion analysis of the 437 base pair Taq1 to Nco1 fragment from the amino terminus of the URA3 gene. Figure 3A depicts the Northern hybridization analysis using a nick-translated, GAL7-specific probe. Figure 3B graphically represents the results of said analysis wherein the 110 base pair transcription effector sequence is shown by an open rectangle.

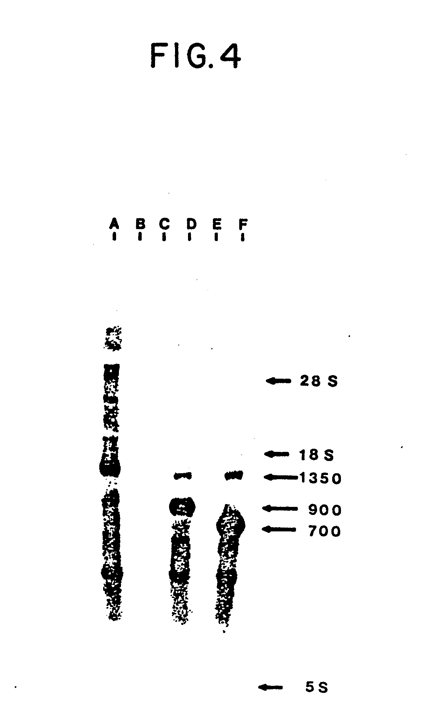

Figure 4 illustrates the effect of orientation of the bifunctional transcription effector sequence upon transcription termination. The northern hybridization shows the results using a nick-translated GAL7-specific probe.

## Detailed Description of the Invention

Unless denoted otherwise in the following discussion, "RNA" refers to messenger RNA (mRNA). All publications cited in this specification are expressly incorporated herein by reference in their entireties.

The approximately 437 base pair Taq1 to Nco1 amino terminal fragment of the URA3 gene of Saccharomyces cerevisiae contains the transcription effector sequence of the present invention. Said 437 base pair fragment of the URA3 gene is present on plasmid pPT4.3 which has been deposited with the American Type Culture Collection and has been given accession number 39850. Plasmid pPT4.3 was constructed from plasmid pBD6-R as shown in Figure 2 and described hereafter. Plasmid pBD6 from which plasmid pBD6-R can be readily prepared has been deposited with the American Type Culture Collection under the designation plasmid pJY6 and has been given accession number 39851. Plasmids pBD6 and pJY6 are identical constructs which were given different laboratory designations.

The 437 base pair Taq1 to Nco1 amino terminal fragment of the URA3 gene has been sequenced and is shown in Table 1.

## TABLE 1

```
          AA  T       20    TT  30        40        50        60
AGCTTTTCTT TGCAATTCAT CATTGGTTTT TTATTCTTTT TTTTGATTTC GGTTTCTTTG
        ••  •                 ••  •

          70        80        90        100       110       120
AAATTTTTTT GATTCGGTAA TCTCCGAACA GAAGGAAGAA CGAAGGAAGG AGCACAGACT
                               ••••• ••••••••••• ••••••••••• •
                                          •••

          130       140       150       160       170       180
TAGATTGGTA TATATACGCA TATGTAGTGT TGAAGAAACA TGAAATTGCC CAGTATTCTT

          190       200       210       220       229
AACCCAACTG CACAGAACAA AAACCTGCAG GAAACGAAGA TAAATCATC TCG AAA GCT
                                                       Met ser lys ala

              250                              ·283
ACA TAT AAG GAA CGT GCT GCT ACT CAT CCT AGT CCT GTT GCT GCC AAG CTA
thr tyr lys glu arg ala ala thr his pro ser pro val ala ala lys leu

              301                              334
TTT AAT ATC ATG CAC GAA AAG CAA ACA AAC TTG TGT GCT TCA TTG GAT GTT
phe asn ile met his glu lys gln thr asn leu cys ala ser leu asp val

              352                              385
CGT ACC ACC AAG GAA TTA CTG GAG TTA GTT GAA GCA TTA GGT CCC AAA ATT
arg thr thr lys glu leu leu glu leu val glu ala leu gly pro lys ile

              403                              436
TGT TTA CTA AAA ACA CAT GTG GAT ATC TTG ACT GAT TTT TCC ATG CGGATCCG
cys leu leu lys thr his val asp ile leu thr asp phe ser met
```

The major methionine start codon for URA3 RNA is shown at base pair 228. The sequence shown in Table 1 differs at five bases from the published sequence of the URA3 gene by Rose and Botstein (J. Molec. Biol., 170: 883-904, 1983). These bases are marked with an asterisk with the published base written above. The Nco1 site was at base 437 while the digested Taq1 site was just prior to base 1. In order to determine the location of the transcription terminator signal(s) within this Taq1 to Nco1 fragment, deletion analysis of this fragment was undertaken as described in detail hereafter. The data generated by this analysis show that the transcription effector sequence is the approximately 110 base pair region between base pair 45 and base pair 155 (denoted in Table 1 by large-type letters). Within this region there is an approximately 15 base pair direct, but overlapping repeat (5' GAACAGAAGGAAGGA) from base pair 86 to base pair 100 and from base pair 98 to base pair 111. Additionally, there is one region of imperfect dyad symmetry from base pair 126 to base pair 140:

TGGTATATATACGCA
ACCATATATATGCGT -5'

As alluded to earlier, this approximately 110 base pair transcription effector sequence is bifunctional in nature. In one aspect, when said sequence is placed downstream (i.e., 3') to a gene encoding a biologically active material, the transcription effector sequence functions to efficiently terminate transcription of said gene. This effect is observed regardless of the orientation of said sequence relative to the gene of interest. In a second aspect, when the transcription effector sequence of the present invention is placed upstream - (i.e., 5') to a gene encoding a biologically active material, transcription of said gene is induced and RNA is synthesized. This induction effect may be manifested in either of two ways. First, the transcription effector sequence may be inserted approximately 20 or more base pairs upstream of the "TATAA box" (i.e., a consensus sequence typically located 10 base pairs upstream of the transcription initiation codon) within a promoter region which lacks a regulatory upstream activation site. Secondly, the transcription induction effect may be manifested by inserting the transcription effector sequence within the promoter region of the gene encoding the biologically active material immediately upstream of the naturally occurring upstream activation site of said gene. Thus, in either of the bifunctional aspects described herein, the transcription effector sequence enhances the expression of the gene encoding the biologically active material. That is to say, the expression of said gene is greater than if the transcription effector sequence of the present invention was not inserted either upstream or downstream of said gene.

By utilizing conventional techniques such as described by Maniatis et al (Molecular Cloning: A Laboratory Manual, Cold Spring Harbor Press, Cold Spring Harbor, New York, 1982) and R.F. Schleif and P.C. Wensink (Practical Methods in Molecular Biology, Springer-Verlag Publishers, New York, 1981), plasmid pPT4.3 was prepared as follows. (All restriction enzymes, T4 DNA ligase, E. coli DNA polymerase, Klenow fragment and synthetic linkers are commercially available from New England Biolabs or Boerhinger-Mannheim.) Plasmid pBD6-R was digested with BamH1 and Sal1 restriction endonucleases thereby generating a 1.7 kilobase (Kb) fragment. Said fragment was then ligated into the BamH1 and Sal1 sites of pBR322 resulting in the plasmid referred to as pPT-1. Plasmid pPT-1 was digested with Taq1 (partial digest) resulting in a 1.4 kb fragment which was isolated and made blunt-ended with Klenow enzyme. This 1.4 kb blunt-end fragment was then ligated, in the presence of HindIII linkers, into the blunt-ended HindIII site of plasmid pBR322-B⁻ (i.e., pBR322 in which the BamH1 site had been removed) thereby producing plasmid pPT2/3. Plasmid pPT2/3 was digested with Nco1 restriction enzyme, made blunt-ended with Klenow enzyme, and recircularized in the presence of BamH1 linkers and ligase thereby producing plasmid pPT4. Plasmid pPT4 was then digested with Cla1 and Bal31 restriction enzymes which removed both the Cla1 and HindIII sites most proximal to Cla1 to produce, after ligation, plasmid pPT4.b. Plasmid pPT4.b was then digested with HindIII, made blunt-ended with Klenow enzyme and then re-ligated in the presence of BamH1 linkers to form plasmid pPT4.3 carrying a 437 base pair sequence bounded by BamH1 restriction sites within which is located the transcription effector sequence of the present invention.

In order to determine the relative location of said transcription effector sequence within the 437 base pair Taq1 to Nco1 fragment of the URA3 gene the following deletion analysis was undertaken according to the general procedure of Maniatis et al, supra. The sequence under analysis (i.e., the 437 base pair Taq1 to Nco1 fragment of the URA3 gene in the BamH1 linker form of plasmid pPT4.3) was ligated into the unique GAL7 BamH1 site in plasmid pBD6 and subsequently referred to as plasmid GA11. The same fragment was also ligated into the indentical site of plasmid pBD6 but said fragment was placed in pBD6 in the opposite orientation. The resulting plasmid construct was referred to as plasmid GA23. In both plasmid GA11 and GA23, deletions were created in vitro by digestion with Bal31 nuclease (commercially available from New England Biolabs or Boerhinger-Mannheim) from the unique Cla1 site of the lacZ gene. After Bal31 digestions, ligations were performed in the presence of Cla1 linkers to mark each deletion endpoint. In all cases, the ends of the deletions were determined by DNA sequencing according to the general method of A. Maxam and W. Gilbert, Proc. Natl. Acad. Sci. USA, 74:560-564 (1977).

Plasmids GA11 and GA23 were then used to transform Saccharomyces cerevisiae (strain DBY745, i.e., α, adel, ura 3-52, leu 2-100, leu 2-112) as follows. 100 milliters (ml) of yeast cells were grown to a concentration of $2 \times 10^7$ cells/ml. The cells were harvested and washed once with 5 ml of TE (i.e., 10 m M Tris, pH 67.5, 1mM EDTA) and once with 5 ml of lithium acetate (LioAc) solution (10 mM Tris, pH 7.5, 1 mM EDTA, and 0.1 M LioAc). The cell pellet was suspended in 1 ml of LioAc solution and incubated for 1 hour at 30°C. Competent yeast cells were either stored for 2 days (or less) at 4°C or used immediately as follows. 0.1 ml of the above cells were added to 15 microliters (µl) plasmid DNA (≤ 1 µg DNA) plus denatured salmon sperm DNA (15 µl of a 3 mg/ml solution) added as a carrier. After incubation for 30 minutes at 30°C, 0.7 ml of polyethylene glycol (PEG) solution (40% PEG 3350, 0.1 MLioAc, 10 mM Tris, pH 7.5, and 1 mM EDTA) was added and incubation was continued for 1 hour at 30°C. The cells were heated at 42°C for 5 minutes and pelleted for 4 seconds in an eppindorf microfuge. The cell pellet was washed twice with 0.5 ml of TE followed by centrifugation for 2 seconds. The final cell pellet was resuspended in 0.1 ml of TE and plated onto selective agar plates.

RNA from the yeast cells transformed by the above procedure was extracted as follows. 5 ml of cycloheximide solution was added to 1 liter of cells at $4 \times 10^7$ cells/ml 10 minutes prior to harvesting. After pelleting, the cells were suspended in 3 ml of RNA buffer (i.e., 50 mM Tris, pH 6.8, 2 mM EDTA, 50 µg/ml cycloheximide) and transferred to a 30 ml corex tube. The cells were ruptures by vortexing 6 times for 20 seconds each in the presence of 0.45 millimeter (mm) glass beads. Sodium dodecyl sulfate (0.1 volume, 10% solution) and 0.1 volume of 2 M sodium acetate (NaAc) pH 5.2 were added, followed by four extractions with a mixture of phenol, chloroform, isoamylalcohol (50:50:1). The final aqueous solution containing the RNA was precipitated by the addition of 0.1 volume of 2 M NaAc, pH 5.2 and 2.5 volumes of 95% ethanol.

RNA extracted by the above procedure was then examined by Northern transfer analysis according to the general method of P. Thomas, Proc. Natl. Acad. Sci. USA, 77:5201-5205 (1980). The Northern filters thus obtained were prehybridized for 2 hours at 42°C in 50% formamide, 5X SSC (1X = .15 M NaCl plus .015 M sodium citrate), 5X Denhardt's solution, 50 mM NaPO₄, pH 6.5, and 200 μg/ml denatured salmon sperm DNA. Hybridization was performed for 12-16 hours at 42°C in a hybridization buffer (i.e., 50% formamide, 5X SSC, 20 mM NaPO₄, pH 6.5, 10% dextran sulfate, 1X Denhardt's solution, 100 μg/ml denatured salmon sperm DNA) with a ³²P-labelled nick-translated probe having a specific activity of 2 X 10⁵ counts per minute/ml hybridization buffer. Said probe was prepared as follows by the general method of T. Maniatis et al, supra. The probe was obtained by cutting plasmid pBD6-Xho with Xho1 and BamH1 nucleases (the Xho1 site is inserted by conventional techniques and is located 4 base pairs in front of the GAL7 ATG codon in this plasmid, while the BamH1 site is located 500 base pairs downstream of the GAL7 ATG codon at the GAL7-lacZ fusion junction) to render a probe specific for the GAL7 coding region. Following hybridization, the filters were washed two times at room temperature for 5 minutes in 2X SSC and 0.1% sodium dodecyl sulfate. The filters were then washed an additional two times for 20 minutes each at 42°C in 0.2X SSC and 0.1% sodium dodecyl sulfate. The filters were then exposed to film at -70°C with Dupont Lightening Plus intensifying screens. The resulting autoradiographs were examined for the presence or absence of a 700-nucleotide GAL7 RNA transcript (from plasmid GA23 deletions) and for a 900-nucleotide GAL7 RNA transcript (from plasmid GA11 deletions). The results of these analyses are shown in Figure 3.

In Figure 3A, lanes A through E correspond, respectively, to RNA extracted from yeast cells containing plasmid GA23 (untreated), GA23Δ300, GA23Δ265, GA23Δ284 and GA23Δ437. Lanes F through J correspond, respectively, to RNA extracted from yeast cells containing plasmid GA11 (untreated), GA11Δ1, GA11Δ10, GA11Δ41, and GA11Δ220. Lane K is pBD6 control RNA. As can be seen from Figure 3A, there is no change in termination patterns for deletions extending up to GA11Δ41 (i.e., a 41 base pair deletion within the fragment) from one orientation and up to GA23Δ284 (i.e., a 284 base pair deletion within the fragment) from the other. However, additional deletions, e.g., GA11Δ220 and GA23Δ437 (Figure 3A, lanes J and E, respectively) eliminated the transcription termination activity. These data indicate that the ability to terminate transcription is dependent upon a 110 base pair region between base pairs 40 and 155 within the 437 base pair Taq1 to Nco1 sequence of the yeast URA3 gene (Figure 3B). Significantly these data prove that the transcription effector sequence of the present invention efficiently terminates transcription when placed downstream (i.e., 3') to a gene encoding a biologically active material. In this instance, GAL7 RNA transcripts were terminated by said sequence as shown in Figure 3.

Further deletion analysis of the 110 base pair transcription effector sequence was performed by constructing synthetic oligomers of portions of said sequence on an Applied Biosystems DNA synthesizer. Deletion 4 (Δ4) of plasmid GA23 was a DNA fragment of the region from base pair 86 through base pair 155 (see Table 1). Deletion 6 (Δ6) was a DNA fragment from base pair 105 through base pair 155 (see Table 1). Deletion 10 (Δ10) was a DNA fragment of plasmid GA11 of the region from base pair 155 through base pair 115 (see Table 1). When tested for the ability to terminate transcription (as described above in the analysis of yeast RNA extracts) deletion 4 showed complete terminator activity whereas deletion 6 and deletion 10 showed no terminator activity. These data suggest that the signal for termination activity may reside at least partly within the region from base pair 85 to base pair 115.

In order to assess the ability of the transcription effector sequence to function in either orientation the following was conducted. Yeast Saccharomyces cerevisiae (strain DBY745) containing either GA11 or GA23 plasmids were grown in MVA medium containing either glucose (uninduced, i.e., no GAL7 transcription) or galactose (induced, i.e., GAL7 transcription present). MVA medium contains 6.7 grams (g) per liter (l) of yeast nitrogen base without amino acids, 10 g/l succinic acid, 6 g/l sodium hydroxide supplemented with adenine and leucine (see J. Yarger et al, Molecular and Cellular Biology, Vol. 6, No. 4, 1986, in press). Total yeast cellular RNA was isolated as described previously and was fractionated by electrophoresis on a formalde-hyde-agarose gel according to the procedure of Yarger et al, Mol. Cell Biochem., 61:173-182 - (1984). The fractionated RNA was then transferred to a nitro-cellulose filter and hybridized to the 500 base pair ³²P-nick-translated DNA probe described above specific for the GAL7 coding region. As shown in Figure 4, when the cells were grown on galactose (lanes A, D and F), the probe DNA hybridized to the 1,350 nucleotide genomic GAL7 RNA. Additionally, cells that contained plasmid GA11 showed a strongly hybridizing 900 nucleotide RNA and those cells containing the plasmid GA23 showed a strongly hybridizing 700 nucleotide RNA. Lanes B, C and E represent those cells grown on glucose (i.e., uninduced). Therefore, the 1,350, 900 and 700 nucleotide transcripts appeared only when galactose was the carbon source (the migration patterns identified in Figure 4 as 28s and 18s are ribosomal RNA, while the 5s is transfer RNA). The GA11 and GA23 RNA transcripts were polyadenylated as determined by oligo(dT) cellulose chromatog-

6

raphy by the method of H. Aviv and P. Leder, Proc. Natl. Acad. Sci. USA, 69:1408-1412 (1972). Also that said transcripts were polyadenylated was evidenced by the fact that GA11 and GA23 RNA transcripts served as good templates for cDNA synthesis when primed with oligo(dT). Thus, the transcription effector sequence of the present invention functions efficiently as a *bona fide* transcription terminator regardless of the orientation thereof when placed 3′ relative to a gene encoding a biologically active material.

To show that the transcription effector sequence of the present invention serves to enhance expression of a gene encoding a biologically active material when placed 3′ to said gene the following was conducted. The transcription effector sequence was placed upstream of the gene encoding a specific protease inhibitor by utilizing conventional techniques thereby forming a recombinant vector. Said vector was then transformed into Saccharomyces cerevisiae and the resulting host cell was cultured. The expression level for the protease inhibitor was approximately 2-fold greater when the transcription effector sequence was present 3′ to the gene for said protease inhibitor than when the transcription effector sequence was not present in the recombinant vector.

As described earlier, the transcription effector sequence of the present invention may be used for enhancing the expression of a gene encoding a biologically active material such as insulin (or proinsulin), human or bovine growth hormones, interferon, blood coagulation factors, chymosin and other enzymes of commercial importance, protease inhibitors, somatostatin, thymosin, antibodies and the like. To achieve such enhanced expression, the transcription effector sequence is placed into a vector containing the gene encoding the biologically active material thereby creating a recombinant vector. The position of the transcription effector sequence in said vector may be upstream or downstream of said gene provided that the gene and the transcription effector sequence are in a functional relation to one another, i.e., that said transcription effector sequence is in a relative juxtaposition to the gene of interest so as to enhance expression thereof as described previously. Such functional location of the transcription effector sequence will be self-evident to those skilled in the art. The recombinant vector may then be used to transform a suitable host cell, typically a eucaryotic host cell. Preferably, the eucaryotic host cell will be a yeast, most preferably Saccharomyces cerevisiae.

As used herein when describing the transcription effector sequence of the present invention, the phrase "functional subunits and derivatives thereof" refers to certain nucleotide sequence(s) within the approximately 110 base pair transcription effector sequence which may exhibit properties ascribed herein to said transcription effector sequence. That is to say, a nucleotide sequence less that 110 base pairs may exist within the transcription effector sequence which is responsible for the activities described herein. That such sequence is not at this time identified (if indeed such exists) does not serve to remove it from the scope and spirit of the present invention if subsequently identified. Further, that synthetic oligomers may be added 5′ or 3′ of the transcription effector sequence to form derivatives thereof, does not serve to remove such derivatives from the ambit of the present invention.

Similarly, the invention described in detail herein is a species of a broader, more generic concept. That is, it has been discovered that the transcription effector sequence of the present invention serves as a binding site for a URA3 regulatory (DNA binding) protein. That such regions exist for other inducible eucaryotic genes is highly probable, said regions having activities such as those ascribed herein to the transcription effector sequence of the present invention. Accordingly, any of such regions are deemed to be contemplated equivalents to the transcription effector sequence of the present invention.

## Claims

1. A transcription effector sequence comprising an approximately 110 base pair region of the yeast URA3 promoter region residing between bases 45 and 155 upstream of the URA3 primary transcription initiation codon located at base 227, and functional subunits and derivatives thereof.

2. A transcription effector sequence comprising the following nucleotide sequence:

5′    GATTTCGGTTTCTTTGAAATTTTTTT

    GATTCGGTAATCTCCGAACAGAAGGAA

    GAACGAAGGAAGGACCACAGACTTAGATT

    GGTATATATACGCATATGTAGTGTTGAAG    3′

and functional subunits and derivatives thereof.

3. A method for enhancing the expression of a gene encoding a biologically active material comprising:

a) introducing the transcription effector sequence of Claims 1 or 2 into a vector containing the gene encoding said biologically active material thereby creating a recombinant vector;

b) transforming a suitable host cell with the recombinant vector of step (a); and

c) culturing said transformed host cell to effect expression of said biologically active material.

4. An expression vector comprising the transcription effector sequence of Claims 1 or 2 and a gene encoding a biologically active material.

5. A host microorganism containing the vector of Claim 4.

6. The host microorganism of Claim 5 which is a yeast.

7. The host microorganism of Claim 6 which is <u>Saccharomyces</u> <u>cerevisiae.</u>

8. Plasmid pPT4.3 having American Type Culture Collection accession number 39850.

9. Plasmid pBD6 having American Type Culture Collection accession number 39851.

# FIG.I

# FIG.2

0 237 913

# FIG.3

A

B

GA23-Δ265 ————————→ +

GA23-Δ284 ————————→ +

GA23-Δ437 ————————→ −

a. LacZ  437 ————————————— 0  Gal 7 →
         400   300   200   100

b. ← Gal 7 ——————————————— LacZ
                                + ← GAII-Δ41

                − ← —————— GAII-Δ220

# FIG.4

A B C D E F

| | **DOCUMENTS CONSIDERED TO BE RELEVANT** | | EP 87103385.8 |
|---|---|---|---|

| Category | Citation of document with indication, where appropriate, of relevant passages | Relevant to claim | CLASSIFICATION OF THE APPLICATION (Int. Cl.4) |
|---|---|---|---|
| D,A | JOURNAL OF MOLECULAR BIOLOGY, vol. 170, 1983 (London, New York, San Francisco)<br><br>M. ROSE et al. "Structure and Function of the Yeast URA 3 Gene; Differentially Regulated Expression of Hybrid β-Galactosidase from Overlapping Coding Sequences in Yeast"<br>pages 883-904<br><br>* Pages 883, 884 *<br>-- | 1 | C 12 N 15/00<br>C 12 N 1/16<br>C 12 N 1/18<br>//(C 12 N 1:18;<br>C 12 R 1:865) |
| D,A | EP - A2 - 0 164 556 (CHIRON CORPORATION)<br><br>* Abstract *<br>-- | 1 | |
| D,A | CELL, vol. 28, no. 3, March 1982 (Cambridge, Mass., USA)<br><br>K.S. ZARET et al. "DNA Sequence Required for Efficient Transcription Termination in Yeast"<br>pages 563-573<br><br>* Totality *<br>-- | 1 | **TECHNICAL FIELDS SEARCHED (Int. Cl.4)**<br><br>C 12 N |
| D,A | EP - A2 - 0 154 566 (TAISHO PHARMACEUTICAL CO. LTD.)<br><br>* Abstract *<br>---- | 1 | |

The present search report has been drawn up for all claims

| Place of search | Date of completion of the search | Examiner |
|---|---|---|
| VIENNA | 10-06-1987 | WOLF |

EPO Form 1503 03 82